# EUROPÄISCHE PATENTANMELDUNG

(11) **EP 0 590 361 A1**
(43) Veröffentlichungstag der Anmeldung: **06.04.1994**
(21) Anmeldenummer: 93114383.8
(22) Anmeldetag: 08.09.1993
(51) Int. Cl.: C07D 473/40

(54) **Verfahren zur Herstellung von 2-Amino-6-halogenpurinen**

(30) Priorität: 17.09.1992 DE 4231036
(71) Anmelder: BASF Aktiengesellschaft, D-67063 Ludwigshafen (DE)
(72) Erfinder: Hagen, Helmut, Dr., D-6710 Frankenthal (DE); Raatz, Peter, Dr., D-6700 Ludwigshafen (DE)

(57) **Zusammenfassung**

Herstellung von 2-Amino-6-halogenpurinen I 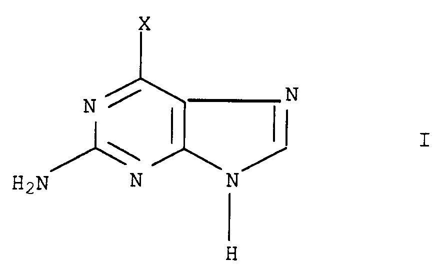
(X = Halogen) durch Hydrolyse von 2-Acylamino-6-halogenpurinen II 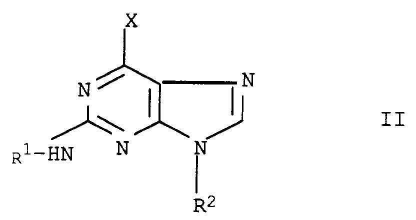
(R¹, R² = Acyl; R² = H) in Gegenwart einer Säure oder Base sowie Herstellung von II durch Acylierung von Guanin III zu Acylguanin Va bzw. Vb 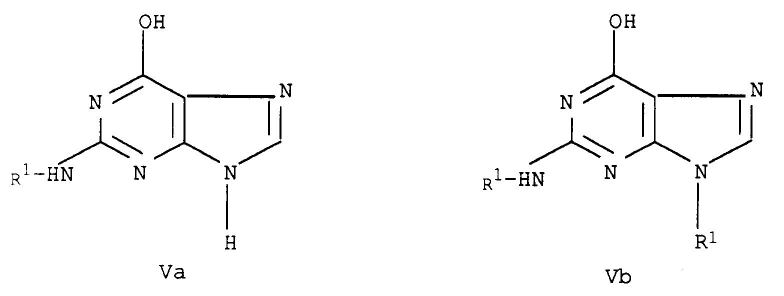
und Halogenierung von V mit einem Halogenierungsmittel VI zu II.

V und II sind neue Zwischenprodukte für die Synthese von I, wenn R¹ und R² Halogenacetyl bedeutet.

## Beschreibung

Die vorliegende Erfindung betrifft neue Verfahren zur Herstellung von 2-Amino-6-halogenpurinen der Formel I 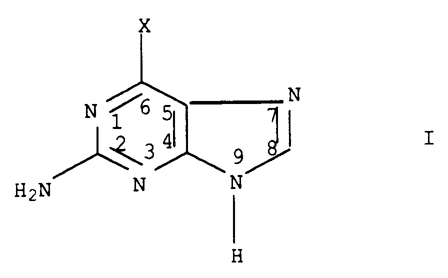
in der X Halogen bedeutet.

Außerdem betrifft die Erfindung neue Acylguanine der allgemeinen Formeln Va und Vb 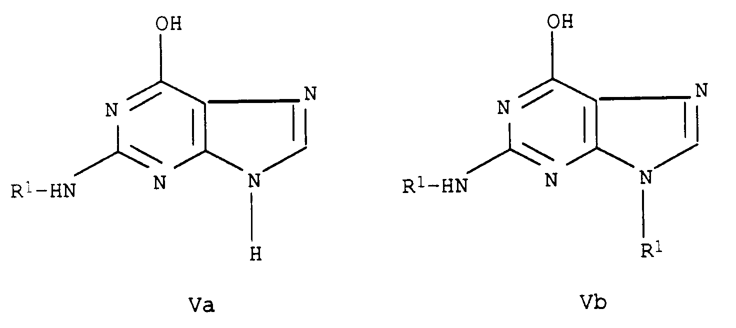
und ihre tautomeren Formen, in der R¹ für eine Halogenacetylgruppe steht, sowie neue 2-Acylamino-6-halogenpurine der allgemeinen Formel II 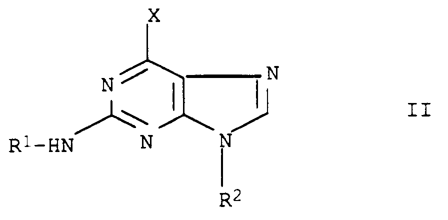
in der R¹ für eine Halogenacetylgruppe und R² für Wasserstoff oder eine Halogenacetylgruppe stehen.

Purine, insbesondere 2-Amino-6-halogenpurine sind allgemein bekannte Verbindungen und dienen als wertvolle Ausgangssubstanzen für die Synthese von Purinnucleosiden, welche ihrerseits für die Herstellung von Pharmazeutika mit Antitumorwirkung von Bedeutung sind.

Aus J. Heterocycl. Chem. 11,77 (1974) ist es bekannt, 2-Amino-6-chlorpurin auf zwei Wegen jeweils ausgehend von Guanin (2-Amino-6-hydroxypurin) in mehreren Verfahrensschritten herzustellen. Wie aus dem dort auf Seite 77 abgebildeten Schema zu entnehmen ist, werden die Zwischenstufen 2-Amino-6-methylthiopurin und Thioguanin jeweils mit Chlor in Gegenwart eines Alkanols bzw. in Gegenwart von Acetonitril oder Salzsäure zu 2-Amino-6-chlorpurin umgesetzt. Die direkte Chlorierung von Guanin mit Chlor ist hiernach nicht möglich.

Nach der EP-A 203 685 gelingt die direkte Chlorierung von Guanin mit Phosphoroxychlorid durch Phasentransferkatalyse in Gegenwart von Tetraethylammoniumchlorid und Acetonitril zu 2-Amino-6-chlorpurin, jedoch ist es hierbei von Nachteil, daß man das 2-Amino-6-chlorpurin nur in geringen Ausbeuten erhält.

Weiterhin ist aus der DE-A 3 941 657 die direkte Chlorierung des durch Acylgruppen geschützten Guanins mittels Phasentransferkatalyse bekannt. So wird 2-Acetylamino-6-hydroxy-9-acetylpurin mit Phosphoroxychlorid in Gegenwart von Tetraethylammoniumchlorid und Triethylamin zu 2-Acetylamino-6-chlor-9-acetylpurin umgesetzt, welches anschließend ohne Isolierung zu 2-Acetylamino-6-chlorpurin aufgearbeitet wird. Die Abspaltung der Acetylgruppe in 2-Stellung ist unter diesen Bedingungen nicht zu beobachten.

Es ist allgemein bekannt, durch Acylgruppen geschützte Aminofunktionen unter sauren und basischen Bedingungen zu hydrolysieren. Als Methode zur Hydrolyse von Acylaminopurinen zu den entsprechenden Aminopurinen ist die Abspaltung der Acylgruppen in Gegenwart von Basen bekannt.

So beschreiben Bowles et al. (J. Med. Chem. 6, 471 (1963)) die Hydrolyse eines in 9-Stellung substituierten 2-Acetylamino-6-hydroxypurins in Gegenwart einer Natriumhydroxydlösung.

Matsumoto et al. (Chem. Pharm. Bull. 36, 1153 (1988)) verwenden in Methanol gelöstes Natriummethylat, um ein in 9-Stellung substituiertes 2-Acetylamino-6-hydroxypurin bei Raumtemperatur zu dem entsprechenden 2-Amino-6-hydroxypurin zu hydrolysieren.

Aus Chem. Pharm. Bull. 37, 336 (1989) ist die Hydrolyse von in 9-Stellung substituiertem 2-Acetylamino-6-chlorpurin in Gegenwart einer Lösung von Natriummethylat in Methanol/Thioethanol bekannt, wobei jedoch neben der hydrolytischen Spaltung der Acetylaminofunktion auch die Dechlorierung stattfindet und somit ein 2-Amino-6-hydroxypurin erhalten wird.

Ferner wird in J. Chem. Soc. Perk. Trans. 2777 (1988) die Umsetzung eines in 9-Stellung substituierten 2-Amino-6-chlorpurins in Gegenwart von Salzsäure zu den entsprechenden 2-Amino-6-hydroxypurin beschrieben.

Da 2-Amino-6-halogenpurine I zu einer Gruppe wichtiger Arzneimittel führen, lag der Erfindung die allgemeine Aufgabe zugrunde, ein besseres und wirtschaftlicheres Gesamtverfahren zur Herstellung von I ausgehend von einfach zugänglichem und damit preiswertem Guanin III unter Verwendung der Zwischenprodukte V und II bereitzustellen.

Eine weitere Aufgabe der Erfindung waren ferner solche Acylguanine Va und Vb und ihre tautomeren Formen, in denen R¹ für eine Halogenacetylgruppe steht, sowie solche 2-Acylamino-6-halogenpurine II, in denen R¹ für eine Halogenacetylgruppe und R² für Wasserstoff oder eine Halogenacetylgruppe stehen.

Speziell erstreckt sich die Aufgabe der Erfindung auf ein neues Verfahren zur Herstellung von 2-Amino-6-halogenpurinen I unter Verwendung von II.

Demgemäß wurde ein neues Verfahren zur Herstellung von 2-Amino-6-halogenpurinen der Formel I 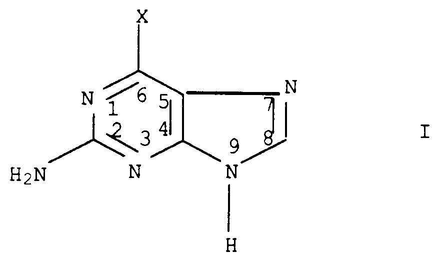
gefunden, in der X Halogen bedeutet, welches dadurch gekennzeichnet ist, daß man 2-Acylamino-6-halogenpurine der Formel II 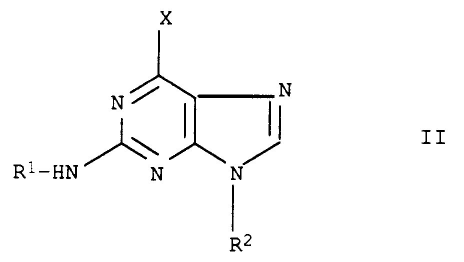
in der R¹ eine Acylgruppe und R² Wasserstoff oder eine Acylgruppe bedeuten, in Gegenwart einer Säure oder Base hydrolysiert.

Im Hinblick auf die Herstellung der für die Arzneimittelsynthese wichtigen 2-Amino-6-halogenpurine I ausgehend von Guanin III kommt dem erfindungsgemäßen Verfahren besondere Bedeutung zu. Darin, daß I in hohen Ausbeuten und somit auf wirtschaftliche Weise durch Hydrolyse von II unter Erhaltung der 6-Halogengruppe zugänglich ist, liegt ein wesentlicher Vorteil des gesamten Verfahrens, welches dadurch gekennzeichnet ist, daß man
a) die 2-Aminogruppe und gewünschtenfalls die 9-NH-Gruppe von Guanin III 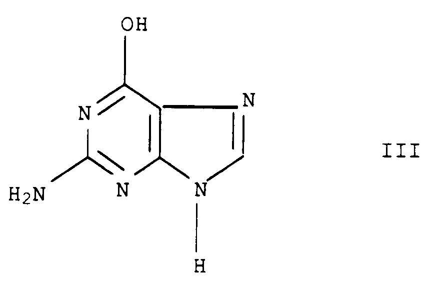 in an sich bekannter Weise mit einem Acylierungsmittel IV zu dem entsprechenden N²-Monoacyl- bzw. N²,9-Diacylguanin der allgemeinen Formel Va bzw. Vb 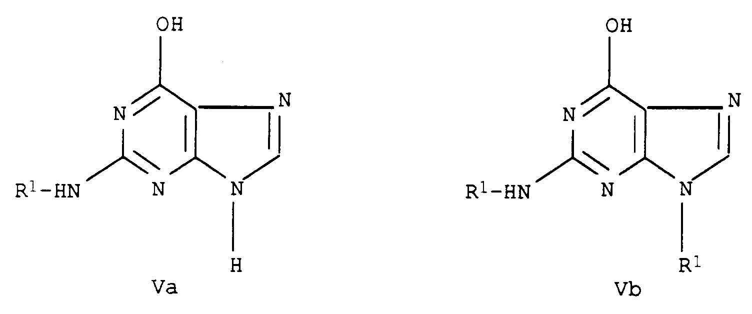 in denen R¹ eine Acylgruppe bedeutet, acyliert,
b) die Acylguanine V mit einem Halogenierungsmittel VI zu 2-Acylamino-6-halogenpurinen II umsetzt und
c) diese danach in Gegenwart einer Säure oder Base zu I hydrolysiert.
   Außerdem wurden als neue Zwischenprodukte die bereits definierten Acylguanine Va und Vb sowie solche 2-Acylamino-6-halogenpurine II, in denen R¹ für eine Halogenacetylgruppe und R² für Wasserstoff oder eine Halogenacetylgruppe stehen, gefunden.
   Das nachfolgende Reaktionsschema veranschaulicht die Herstellung von 2-Amino-6-halogenpurinen I in drei Verfahrensschritten ausgehend von Guanin III:

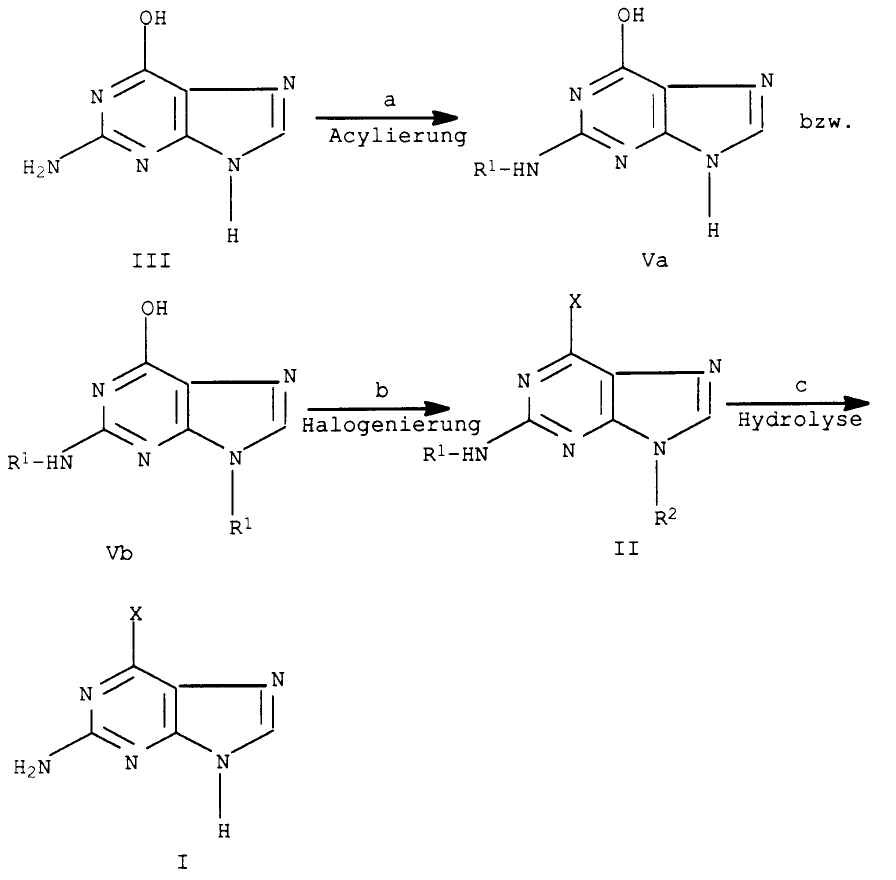

### Hydrolyse

Die Herstellung von I nach Anspruch 1 fügt sich vorteilhaft in das Gesamtverfahren ein, insbesondere dann, wenn X für die Substituenten Chlor und Brom steht.

Die Hydrolyse von II wird (Verfahrensschritt (c)) in Gegenwart einer Säure oder einer Base durchgeführt.

Als Säuren kommen neben sauren Ionentauschern Mineralsäuren wie Salzsäure, Schwefelsäure und Phosphorsäure sowie aliphatische und aromatische Carbonsäuren wie Ameisensäure, Essigsäure, Propionsäure und Benzoesäure in Betracht. Ferner kann p-Toluolsulfonsäure verwendet werden. Besonders bevorzugt ist Salzsäure.

Als Basen kommen neben Hydroxiden und Carbonaten vorzugsweise Alkoholate in Betracht. Die Alkoholate leiten sich von folgenden Alkoholen wie
Alkanolen, z. B. Methanol, Ethanol, n-Propanol, Isopropanol, n-Butanol, Isobutanol, sek.-Butanol, tert.-Butanol, n-Pentanol, 2-Methylbutanol und 3-Methylbutanol,
Cycloalkanolen, z. B. Cyclopropanol, Cyclobutanol, Cyclopentanol und Cyclohexanol, sowie
araliphatischen Alkoholen, z. B. Benzylalkohol und 2-Phenylethanol, ab.
Bevorzugt werden Alkanole mit 1 bis 4 C-Atomen verwendet, insbesondere Ethanol, Isopropanol und Isobutanol.

Der kationische Teil der Base ist vorzugsweise ein Alkalimetall wie Lithium, Natrium, Kalium und Rubidium, insbesondere Natrium. Daneben eignen sich Erdalkalimetalle wie Magnesium und Calcium.

Genannt seien beispielsweise
Hydroxide wie Natrium- und Kaliumhydroxid,
Carbonate wie Kalium- und Magnesiumcarbonat sowie
Alkoholate wie Natriumethylat, -n-propanolat und -iso-butanolat, wobei Natriumethylat besonders bevorzugt ist.

Die Menge an Base liegt in der Regel bei 0,5 bis 5, vorzugsweise 1 bis 2 mol pro Mol II. Für den Fall, daß die Hydrolyse von II in Gegenwart einer Säure durchgeführt wird, ist es zweckmäßig, die Säure in geringem Überschuß über die stöchiometrisch erforderliche Menge zu verwenden. Es empfiehlt sich, pro Mol II 1 bis 2, vorzugsweise 1,2 bis 1,5 mol Säure einzusetzen, wobei der über die stöchiometrische Menge hinausgehende Anteil Säure katalytisch wirkt.

Die Säure wird üblicherweise in Form einer wäßrigen Lösung verwendet, wobei sie in einer Konzentration von 1 bis 20, bevorzugt 3 bis 10 Gew.-% im Lösungsmittel vorliegt.

Neben Wasser sind als weitere Lösungs- und Verdünnungsmittel - insbesondere bei Verwendung einer Base - inerte organische Flüssigkeiten wie Tetrahydrofuran, Dioxan, Acetonitril, N,N-Dimethylformamid, N-Methylpyrrolidon, 1,2-Dialkoxyethane wie 1,2-Dimethoxyethan und 1,2-Diethoxyethan sowie Alkohole, z. B. Methanol, Ethanol, n-Propanol, Isopropanol, n-Butanol, Isobutanol, Cyclohexanol und Benzylalkohole geeignet. Erfolgt die Abspaltung der Acylgruppen von II in Gegenwart eines Metallalkoholates, so empfiehlt sich ein dem Alkoholat entsprechender Alkohol. Die Menge des Lösungsmittels liegt im allgemeinen zwischen 10 bis 50 kg pro kg II.

Die Umsetzung erfolgt bei einer Reaktionstemperatur von 0 bis 120°C, vorzugsweise 70 bis 100°C. Höhere Temperaturen sind von Vorteil, da so die Reaktionszeiten verkürzt werden und infolgedessen die als nicht erwünschte Nebenreaktion ablaufende Abspaltung der 6-Halogengruppe zurückgedrängt wird.

Die Reaktionszeiten können je nach Reaktionstemperatur bis zu 6 Stunden betragen, wobei eine Dauer von 5 bis 30 Minuten in der Regel zweckmäßig ist.

Das gute Gelingen des erfindungsgemäßen Verfahrens ist von der Art der Substituenten X, R¹ und R² der Ausgangsverbindung II nicht erkennbar abhängig. Als II kommen neben den in 6-Stellung durch Fluor, Brom und Jod substituierten Substanzen insbesondere 2-Acylamino-6-chlorpurine in Betracht. Die weiteren Reste R¹ und R² bedeuten jeweils eine Acylgruppe, wobei R² außerdem noch für Wasserstoff steht.

Unter der Bezeichnung Acylgruppe sind die Reste von beliebigen Carbonsäuren zu verstehen, beispielsweise von aliphatischen und cycloaliphatischen Carbonsäuren wie
Essigsäure,
Propionsäure,
Buttersäure,
i-Buttersäure,
n-Valeriansäure,
i-Valeriansäure,
Hexansäure,
Heptansäure,
Octansäure,
Nonansäure,
Cyclopentancarbonsäure und
Cyclohexancarbonsäure,
von araliphatischen Carbonsäuren wie
Benzoesäure und
Phenylessigsäure sowie
von halogenierten aliphatischen Carbonsäuren wie
Monofluor-, Difluor- und Trifluoressigsäure,
Monochlor-, Dichlor- und Trichloressigsäure,
Monobrom-, Dibrom- und Tribromessigsäure, wobei auch Halogenpropionsäuren, Halogenbuttersäuren und Halogenvaleriansäuren in Betracht kommen.

Im Verfahrensschritt (c) geht man zur Herstellung der Verbindungen I bevorzugt von folgenden 2-Acylamino-6-halogenpurinen II aus:
2-Acetylamino-6-chlorpurin,
2-Monochloracetylamino-6-chlorpurin,
2-Dichloracetylamino-6-chlorpurin,
2-Trichloracetylamino-6-chlorpurin,
2-Monochloracetylamino-6-chlor-9-monochloracetylpurin, wobei 2-Acetylamino-6-chlorpurin und die 2-Chloracetylamino-6-chlorpurine besonders bevorzugt sind.

Das erfindungsgemäße Verfahren kann sowohl diskontinuierlich als auch kontinuierlich durchgeführt werden. Bei der diskontinuierlichen Arbeitsweise werden Ausgangssubstanz II und Säure oder Base zusammen mit dem Lösungs- und Verdünnungsmittel vorgelegt und bei der gewählten Temperatur umgesetzt. Außerdem kann II zusammen mit dem Lösungs- und Verdünnungsmittel vorgelegt werden, und Säure oder Base können danach in die auf Reaktionstemperatur gebrachte Lösung eingeleitet werden. Arbeitet man nach der kontinuierlichen Methode, so kann die Ausgangssubstanz II sowohl in gelöster als auch suspendierter Form entweder zusammen mit der Säure oder Base oder getrennt über 2 zwei Zuleitungen in den Reaktor eingeleitet werden. Die umgesetzte Mischung wird mit der gleichen Geschwindigkeit aus dem Reaktor entfernt und aufgearbeitet, wie nicht umgesetzte Reaktionsteilnehmer in den Reaktor geleitet werden.

Im übrigen erfolgt die Aufarbeitung des Reaktionsgemisches auf bekannte Weise durch Neutralisation, Filtration, Destillation, Umkristallisation, Digerieren und Trocknung des Produktes I, so daß nähere Angaben hierzu entbehrlich sind.

In der Regel liegen die Ausbeuten an 2-Amino-6-halogenpurinen I bezogen auf eingesetztes II im Bereich von 70 bis 85 %, wobei I im allgemeinen in einer Reinheit von über 90 % erhalten wird.

Wie bereits oben im Reaktionsschema dargestellt, sind für die Herstellung der im Verfahrensschritt (c) als Ausgangssubstanz verwendeten 2-Acylamino-6-halogenpurine II zuvor die Verfahrensschritte Acylierung (a) und Halogenierung (b) erforderlich.

### Acylierung

Im Verfahrensschritt (a) geht man zur Herstellung der Zwischenprodukte V von Guanin III aus und setzt es in an sich bekannter Weise, z. B. H. Hrebabecky, J. Farkas, Nucleic Acid Chemistry, 1978, 13-14, mit einem Acylierungsmittel IV, vorzugsweise dem Anhydrid oder einem Säurehalogenid einer Carbonsäure, wie dem Säurechlorid um. Dabei kommen beispielsweise solche Anhydride und Säurechloride in Betracht, die sich von den oben aufgezählten Carbonsäuren ableiten.

Die Verwendung von Carbonsäureanhydriden als Acylierungsmittel kommt insbesondere dann in Betracht, wenn N²,9-diacylierte Verbindungen (Vb) als Verfahrensprodukte gewünscht sind.

In der Regel erfolgt die Acylierung bei einer Reaktionstemperatur von 0 bis 170°C, vorzugsweise 70 bis 150°C, in Gegenwart von organischen Flüssigkeiten als Reaktionsmedium. Hierzu eignen sich neben N-Methylpyrrolidon, Nitrobenzol und Dimethylformamid diejenigen Carbonsäuren, von denen sich das jeweils gewählte Acylierungsmittel ableitet, z. B. Eisessig, Propionsäure, Buttersäure, Monochloressigsäure, Dichloressigsäure und Trichloressigsäure. Für den Fall, daß bei der Umsetzung keine Flüssigkeit als Lösungs- und Verdünnungsmittel verwendet wird, empfiehlt es sich, die Reaktion in der Schmelze vorzunehmen. Dabei ist es zweckmäßig, das Acylierungsmittel in größeren Mengen als stöchiometrisch erforderlich zu verwenden.

Ferner kann die Umsetzung in Gegenwart einer Base wie einem Alkalimetallsalz einer Carbonsäure, z. B. Natriumacetat, Natriumpropionat, Natriummonochloracetat vorgenommen werden.

Außerdem empfiehlt es sich, insbesondere zur Herstellung der N²,9-diacylierten Verbindungen Vb, die Acylierung unter Ausschluß von Wasser durchzuführen.

### Halogenierung

Im Verfahrensschritt (b) erfolgt die Umsetzung von Acylguaninen V mit einem Halogenierungsmittel VI zu den 2-Acylamino-6-halogenpurinen II.

Man geht dabei sowohl von N²-Monoacylguaninen Va als auch von N²,9-Diacylguaninen Vb, in denen R¹ für eine Acylgruppe, insbesondere für eine Halogenacetylgruppe steht, aus.

Besonders bevorzugte Acylguanine V sind:
2-Acetylamino-6-hydroxypurin,
2-Monochloracetylamino-6-hydroxypurin,
2-Dichloracetylamino-6-hydroxypurin und
2-Trichloracetylamino-6-hydroxypurin und
N²,9-Di-(monochloracetyl)guanin.

Als Halogenierungsmittel VI kommen alle nucleophilen Halogenierungsmittel in Betracht, insbesondere Agenzien für die Chlorierung und Bromierung der 6-Stellung des Purin-Körpers. Genannt seien beispielsweise die üblichen Chlorierungsmittel wie Thionylchlorid, Sulfurylchlorid, Phosgen, Phosphoroxychlorid und Phosphorpentachlorid sowie die üblichen Bromierungsmittel, z. B. Brom, Thionylbromid, Phosphorpentabromid und Phosphoroxybromid. Als Halogenierungsmittel VI verwendet man vorzugsweise Phosphoroxychlorid und Phosphoroxybromid.

Als Fluorierungsmittel kommen Substanzen wie Antimonfluorid und Chlorfluorid in Betracht. Als Jodierungsmittel kann Jod verwendet werden. Ferner ist es möglich, das Chlor- und Bromatom in 6-Stellung von II durch Fluor und Jod auszutauschen.

Es ist zweckmäßig, 1 bis 10 mol, bevorzugt 2 bis 4 mol Halogenierungsmittel VI pro Mol der Verbindung V zu verwenden.

Man führt die Halogenierung der Acylguanine V in Gegenwart einer Base sowie vorzugsweise zusätzlich in Gegenwart eines quartären Ammoniumhalogenids VII aus. Als Basen, welche zur Neutralisation der bei der Umsetzung freiwerdenden Säuren HX dienen, eignen sich Amine wie Triethylamin, Diethylamin, n-Butylamin, N,N-Dimethylanilin, N,N-Diethylanilin, Cyclohexylamin, N-Ethylpiperidin sowie Pyridine wie Pyridin, Picoline und Lutidine, wobei vor allem Triethylamin und N-Ethylpiperidin bevorzugt sind.

Die Menge an Base beträgt in der Regel 0,5 bis 4, vorzugsweise 1 bis 2 mol pro Mol der Verbindung V.

Als quartäre Ammoniumhalogenide VII, welche die Umsetzung von V mit VI katalytisch begünstigen, eignen sich praktisch alle Verbindungen dieses Typs, wobei von Vorteil ist, wenn das Anion X' dem in Verbindung II einzuführenden Halogen X entspricht. Es kommen Chloride und Bromide, insbesondere Chloride in Betracht.

Bevorzugt werden Salze der Formel VIIa 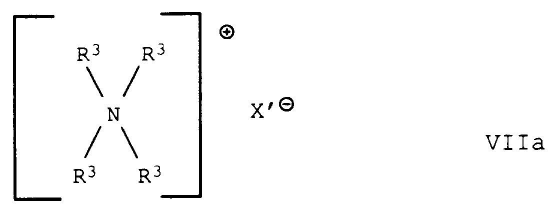
in der die Reste R³ gleiche oder verschiedene aliphatische und cycloaliphatische Gruppen mit 1 bis 16 C-Atomen bedeuten, wobei zwei der Reste R³ auch zu einem Ring, der durch Sauerstoff unterbrochen sein kann, verbunden sein können, und wobei einer dieser Reste auch durch eine unsubstituierte oder substituierte Phenyl-, Benzyl- oder 2-Phenylethylgruppe ersetzt sein kann, und in der X' Chlor, Brom oder Jod bedeutet.

Genannt seien beispielsweise Tetramethylammoniumchlorid, -bromid und -jodid, Tetraethylammoniumchlorid, -bromid und jodid, Tetra-(n-butyl)-ammoniumchlorid, -bromid und jodid sowie vor allem Benzyltriethylammoniumchlorid und -bromid.

Die Menge dieser Katalysatoren ist nicht kritisch, liegt jedoch zur Erzielung wirtschaftlich befriedigender Reaktionsgeschwindigkeiten in der Regel bei 0,5 bis 3 mol pro Mol V. Es empfiehlt sich, VII in geringem stöchiometrischen Überschuß einzusetzen, wobei ca. 1,05 bis 1,3 mol pro Mol V bevorzugt sind.

Die quartären Ammoniumhalogenide VII sind kommerziell erhältlich; sie können aber auch - insbesondere unter dem Aspekt der Wirtschaftlichkeit - aus einem Amin N(R³)₃ und einem Halogenid R³X in an sich bekannter Weise in Gegenwart eines auch für die Halogenierung zweckmäßigen Lösungsmittels hergestellt werden. Dabei ist es verfahrenstechnisch von Vorteil, daß Amin und Base identische Verbindungen sind.

Bei der Umsetzung von V zu II empfiehlt sich für die Reaktionstemperatur der Bereich von 0 bis 100°C, insbesondere 20 bis 50⁰C. Bei tieferen Temperaturen ist die Reaktionsgeschwindigkeit der Umsetzung zu gering, und bei höheren Temperaturen, insbesondere oberhalb von 100°C, ist mit Nebenreaktionen wie Mehrfachhalogenierung von V zu rechnen.

Die Reaktionszeiten liegen im allgemeinen zwischen 30 Minuten und 80 Stunden, wobei 5 bis 50 Stunden üblich sind.

In der Regel führt man die Umsetzung unter Normaldruck oder unter dem leicht erhöhten Eigendruck des Reaktionsgemisches aus, wenn man ein tief siedendes Lösungs- oder Verdünnungsmittel verwendet.

Die Mitverwendung eines Lösungs- oder Verdünnungsmittels ist in den meisten Fällen von Vorteil.

Als Lösungs- und Verdünnungsmittel - man kann sowohl in Lösung als auch in Suspension arbeiten - eignen sich inerte organische Flüssigkeiten wie Tetrahydrofuran, Dioxan, 1,2-Dimethoxyethan und 1,2-Diethoxyethan sowie vor allem Nitrobenzol und Acetonitril. Die Menge der Lösungsmittel liegt im allgemeinen zwischen 1 und 10 kg pro kg V.

Die Halogenierung von V zu II erfolgt im allgemeinen in der Weise, daß zur - im Lösungsmittel vorgelegten - Ausgangssubstanz V eine Base und ein quartäres Ammoniumhalogenid VII gegeben werden. Danach erfolgt die Zugabe des Halogenierungsmittels VI.

Die Aufarbeitung der Reaktionsmischung wird so durchgeführt, daß VI in Gegenwart einer Base, z. B. einer wäßrigen Lösung von Ammoniak, Natrium- und Kaliumhydroxid hydrolysiert wird. Dabei empfiehlt es sich, im schwach sauren pH-Bereich (ca. 5) zu arbeiten. Die weitere Aufarbeitung und Isolierung von II erfolgt nach den üblichen Methoden.

Die im Hinblick auf die Synthese von Purinnucleosiden wichtigen 2-Amino-6-halogenpurine I sind durch die erfindungsgemäßen Verfahren in einer hervorragenden Reinheit und guten Ausbeuten erhältlich. Allgemein bieten die Verfahren und die dazugehörigen Zwischenprodukte V und II die Möglichkeit zur wirtschaftlichen Synthese von Verbindungen I.

### Beispiel 1

### Herstellung von 2-Acetylamino-6-hydroxypurin (Va/1)

151 g (1 mol) Guanin und 172 g (2,1 mol) wasserfreies Natriumacetat wurden in 1,5 l Eisessig vorgelegt und zum Sieden erhitzt. Anschließend wurden 224,4 g (2,2 mol) Essigsäureanhydrid zugetropft. Nach 8-stündigem Erhitzen wurde die Reaktionsmischung abgekühlt, das Reaktionsprodukt wurde abfiltriert und mit Wasser gewaschen. Nach Trocknen wurde das Produkt in einer Ausbeute von 91 % erhalten.
Schmp. > 280°C

### Beispiel 2

### Herstellung von 2-Monochloracetylamino-6-hydroxypurin (Va/2)

Diese Verbindung wurde analog Beispiel 1 aus Guanin und Monochloressigsäureanhydrid in Gegenwart von Monochloressigsäure und ihrem Natriumsalz hergestellt. Nach 2-stündigem Erhitzen wurde die Reaktionsmischung abgekühlt, und das Produkt wurde abfiltriert und getrocknet.
Ausbeute: 84 %
¹H-NMR (D₆-DMSO):
δ (ppm) : 4,44 (2H, N²-CO-CH₂Cl); 8,1 (1H, C⁸-H)

### Beispiel 3

### Herstellung von 2-Dichloracetylamino-6-hydroxypurin (Va/3)

Diese Verbindung wurde analog Beispiel 2 aus Guanin und Dichloressigsäureanhydrid in Gegenwart von Dichloressigsäure und ihrem Natriumsalz hergestellt.
Ausbeute: 52 %
¹H-NMR (D₆-DMSO):
δ (ppm) : 6,68 (1H, N²-CO-CHCl₂); 8,27 (1H, C⁸-H)

### Beispiel 4

### Herstellung von 2-Trichloracetylamino-6-hydroxypurin (Va/4)

Diese Verbindung wurde analog Beispiel 2 aus Guanin und Trichloressigsäureanhydrid in Gegenwart von Trichloressigsäure und ihrem Natriumsalz hergestellt.
Ausbeute: 49 %
¹H-NMR (D₆-DMSO):
δ (ppm) : 8,34 (1H, C⁸-H)

### Beispiel 5

### Herstellung von N²,9-Di-(monochloracetyl-)guanin (2-Monochloracetylamino-6-hydroxy-9-monochloracetylpurin) (Vb/5)

In einem Reaktionsgefäß von 200 l Inhalt wurden 85,5 kg (500 mol) Monochloressigsäureanhydrid bei 70°C unter Rühren aufgeschmolzen. Zu der Schmelze wurden 15,1 kg (100 mol) Guanin gegeben. Nach 2-stündigem Rühren bei 100°C wurde die Reaktionsmischung abgekühlt, wobei ab einer Temperatur von ca. 70°C Tetrahydrofuran zugegeben wurde. Nach 1-stündigem Rühren bei Raumtemperatur wurde das Produkt abfiltriert, mit Tetrahydrofuran gewaschen, wieder abfiltriert und getrocknet.
Ausbeute: 91 %
Schmp. > 250°C
¹H-NMR (D₆-DMSO):
δ (ppm) : 4,5 (2H, N⁹-CO-CH₂Cl); 5,3 (2H, NH-CO-CH₂Cl); 8,56 (1H, C⁸-H)

### Beispiel 6

### Herstellung von 2-Acetylamino-6-chlorpurin (II/1)

Eine Lösung von 82,2 g (0,65 mol) Benzylchlorid und 66,7 g (0,66 mol) Triethylamin in 1,2 l Acetonitril wurde 6 Stunden unter Rückfluß erhitzt. Danach wurden zu der auf ca. 5 bis 10°C abgekühlten Lösung 52,5 g (0,52 mol) Triethylamin und 97 g (0,5 mol) der Verbindung Va/1 gegeben. Weiterhin wurden 307 g (2 mol) Phosphoroxychlorid zugetropft. Die Mischung wurde anschließend 16 Stunden bei Raumtemperatur gerührt und dann so in 1,2 l kaltes Wasser eingeleitet, daß die Temperatur bei ca. 10°C lag. Gleichzeitig wurde 2-molare Natronlauge zugegeben, so daß die Mischung einen pH-Wert von ca. 5 hatte. Unter diesen Bedingungen wurde sie noch eine Stunde gerührt, das ausgefallene Produkt wurde abgetrennt, mit Wasser gewaschen und getrocknet.
Ausbeute: 85 %
Schmp. > 280°C

### Beispiel 7

### Herstellung von 2-Monochloracetylamino-6-chlorpurin (II/2)

Diese Verbindung wurde analog Beispiel 6 aus Verbindung Va/2 und Phosphoroxychlorid hergestellt.
Ausbeute: 81 %
Schmp. > 250°C
¹H-NMR (D₆-DMSO):
δ (ppm) : 4,47 (2H,NH-CO-CH₂Cl; 11,17 (1H, NH-CO-CH₂Cl); 8,56 (1H, C⁸-H)

### Beispiel 8

### Herstellung von 2-Monochloracetylamino-6-chlorpurin (II/2) aus 2-Monochloracetylamino-6-hydroxy-9-monochloracetylpurin (Vb/5)

Zu 130 l Nitrobenzol wurden unter Rühren 13,7 kg (60 mol) Benzyltriethylammoniumchlorid und 5,8 kg (51 mol) N-Ethylpiperidin gegeben. Dann wurden 38,3 kg (250 mol) Phosphoroxychlorid so zugegeben, daß 40°C in der Lösung nicht überschritten wurden. Danach wurden 15,2 kg der Verbindung Vb/5 hinzugefügt. Nach 20-stündigem Rühren bei 40°C wurden die Reaktionsmischung und 16 l konzentrierte wäßrige Ammoniaklösung unter Rühren in 200 l Wasser eingeleitet, wobei 15°C in der Mischung nicht überschritten und ein pH-Wert von ca. 5 eingehalten wurden. Nach 30-minütigem Rühren bei Raumtemperatur und unter Einhaltung des pH-Wertes von ca. 5 wurde das angefallene Reaktionsprodukt abfiltriert.
Ausbeute: 78 %

### Beispiel 9

### Herstellung von 2-Trichloracetylamino-6-chlorpurin (II/3)

Diese Verbindung wurde analog Beispiel 6 aus Verbindung Va/4 und Phosphoroxychlorid hergestellt.
Ausbeute: 35 %
¹H-NMR (D₆-DMSO):
δ (ppm) : 8,72 (1H; C⁸-H) ; 11,91 (1H)

### Beispiel 10

### Herstellung von 2-Amino-6-chlorpurin (I/1) aus II/1 in Gegenwart von Natriumethylat

70 g (0,33 mol) 2-Acetylamino-6-chlorpurin (II/1) wurden mit 25 g (0,37 mol) Natriumethylat in 1 l Ethanol 15 Minuten unter Rühren bei Rückflußtemperatur zur Reaktion gebracht. Nachdem die Reaktionsmischung mit Wasser verdünnt und mit Schwefelsäure neutralisiert worden war, wurde 95 % GC-reines Produkt in einer Ausbeute von 80 % erhalten.

### Beispiel 11

### Herstellung von 2-Amino-6-chlorpurin (I/1) aus II/1 in Gegenwart von Salzsäure

In 1,5 l Wasser wurden 70 g (0,33 mol) 2-Acetylamino-6-chlorpurin (II/1) vorgelegt. Zu der 95°C heißen Mischung wurden innerhalb von 15 Minuten unter Rühren 0,5 l 1,2 normale Salzsäure zugegeben. Anschließend wurde die auf ca. 5°C abgekühlte Reaktionsmischung mit konzentrierter wäßriger Ammoniaklösung neutralisiert. Nach Filtration wurde 93 % reines Produkt in einer Ausbeute von 70 % erhalten.

### Beispiel 12

### Kontinuierliche Herstellung von 2-Amino-6-chlorpurin (I/1) aus II/1 in Gegenwart von Salzsäure

Eine 6 gew.-%ige wäßrige Suspension von 2-Acetylamino-6-chlorpurin (II/1) und 0,6 normale Salzsäure wurden jeweils mit einer Geschwindigkeit von 40 ml/min aus zwei getrennten Vorratsgefäßen in eine Rührkesselkaskade (2 Kessel) aus je 1 l-Gefäßen geleitet und dort bei einer Temperatur von 95°C zur Reaktion gebracht. Dabei betrug die mittlere Verweilzeit 15 Minuten. Im letzten Reaktionsgefäß wurde die auf ca. 5°C abgekühlte Reaktionsmischung mit konzentrierter Ammoniaklösung neutralisiert. Nach Filtration wurde 96 % reines Produkt in einer Ausbeute von 85 % erhalten.

### Beispiel 13

### Kontinuierliche Herstellung von 2-Amino-6-chlorpurin (I/1) in Gegenwart von Salzsäure

2-Amino-6-chlorpurin (I/1) wurde analog Beispiel 12 aus den Verbindungen II/2 und II/3 jeweils durch Hydrolyse in Gegenwart von 0,6 normaler Salzsäure hergestellt. Das Produkt I/1 wurde in folgenden Reinheiten und Ausbeuten erhalten:

| Edukt | Reinheit (%) | Ausbeute % |
|---|---|---|
| II/2 | 97 | 78 |
| II/3 | 92 | 54 |

## Patentansprüche

1. Verfahren zur Herstellung von 2-Amino-6-halogenpurinen der Formel I 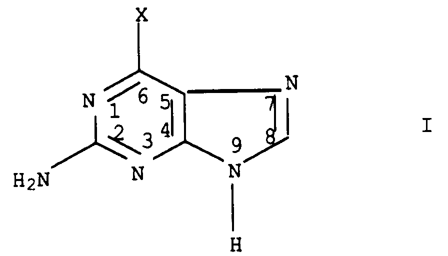 in der X Halogen bedeutet, dadurch gekennzeichnet, daß man 2-Acylamino-6-halogenpurine der Formel II 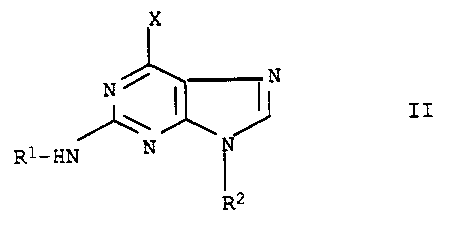 in der R¹ eine Acylgruppe und R² Wasserstoff oder eine Acylgruppe bedeuten, in Gegenwart einer Säure oder Base hydrolysiert.

2. Verfahren nach Anspruch 1, dadurch gekennzeichnet, daß man bei einer Reaktionstemperatur von 0 bis 120°C hydrolysiert.

3. Verfahren zur Herstellung von 2-Amino-6-halogenpurinen der Formel I 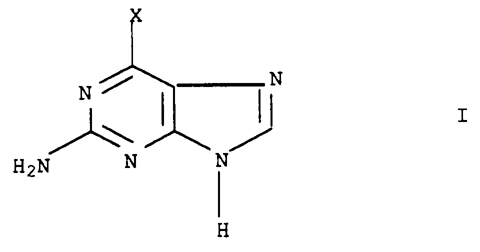 in der X Halogen bedeutet, dadurch gekennzeichnet, daß man
a) die 2-Aminogruppe und gewünschtenfalls die 9-NH-Gruppe von Guanin III 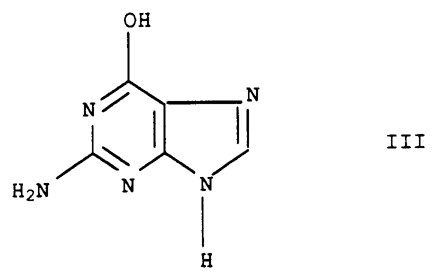 in an sich bekannter Weise mit einem Acylierungsmittel IV zu dem entsprechenden N²-Monoacyl- bzw. N²,9-Diacylguanin der allgemeinen Formel Va bzw. Vb 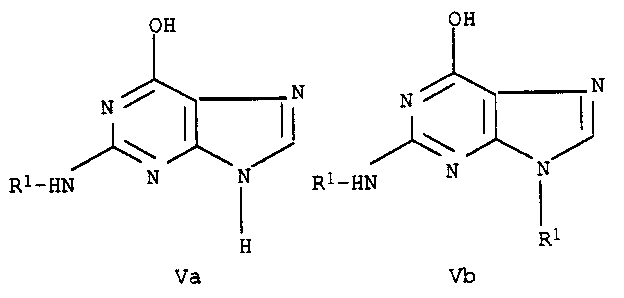 acyliert,
b) die Acylguanine V mit einem Halogenierungsmittel VI zu 2-Acylamino-6-halogenpurinen II umsetzt und
c) diese danach in Gegenwart einer Säure oder Base zu I hydrolysiert.

4. Verfahren nach Anspruch 3, dadurch gekennzeichnet, daß man als Halogenierungsmittel VI Phosphoroxychlorid oder Phosphoroxybromid verwendet.

5. Verfahren nach Anspruch 3 oder 4, dadurch gekennzeichnet, daß man die Verbindungen V in Gegenwart einer Base und eines quartären Ammoniumhalogenids VII mit einem Halogenierungsmittel VI zu II umsetzt.

6. Verfahren nach Anspruch 5, dadurch gekennzeichnet, daß man als quartäres Ammoniumhalogenid VII ein Salz der Formel VIIa 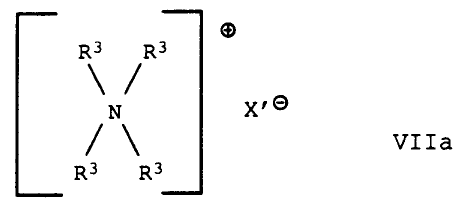 verwendet, in der die Reste R³ gleiche oder verschiedene aliphatische und cycloaliphatische Gruppen mit 1 bis 16 C-Atomen bedeuten, wobei zwei der Reste R³ auch zu einem Ring, der durch Sauerstoff unterbrochen sein kann, verbunden sein können, und wobei einer dieser Reste auch durch eine unsubstituierte oder substituierte Phenyl-, Benzyl- oder 2-Phenylethylgruppe ersetzt sein kann, und in der X' Chlor, Brom oder Jod bedeutet.

7. Verfahren nach den Ansprüchen 5 und 6, dadurch gekennzeichnet, daß man als quartäres Ammoniumhalogenid VII ein Chlorid verwendet.

8. Acylguanine der allgemeinen Formel Va und Vb gemäß Anspruch 3 und ihre tautomeren Formen, in denen R¹ für eine Halogenacetylgruppe steht.

9. Acylguanine nach Anspruch 8, in der R¹ für eine Monochloracetyl-, Dichloracetyl- und Trichloracetylgruppe steht.

10. 2-Acylamino-6-halogenpurine der allgemeinen Formel II gemäß den Ansprüchen 1 bis 3, in der R¹ für eine Halogenacetylgruppe und R² für Wasserstoff oder eine Halogenacetylgruppe stehen.

11. 2-Acylamino-6-halogenpurine nach Anspruch 10, in der R¹ für eine Monochloracetyl-, Dichloracetyl- oder Trichloracetylgruppe und R² für Wasserstoff oder eine Monochloracetylgruppe stehen.
